# EUROPEAN PATENT SPECIFICATION

(11) **EP 2 569 025 B1**
(45) Date of publication and mention of the grant of the patent: **29.07.2015**
(21) Application number: 11728668.2
(22) Date of filing: 12.05.2011
(51) Int. Cl.: A61L 27/50, A61L 24/00, A61L 27/44

(54) **INJECTABLE OSTEOINDUCTIVE BONE CEMENTS**
INJIZIERBARER OSTEOINDUKTIVER KNOCHENZEMENT
CIMENTS OSSEUX OSTÉOINDUCTIFS INJECTABLES

(30) Priority: 13.05.2010 IT TO20100401
(43) Date of publication of application: 20.03.2013
(73) Proprietor: Politecnico di Torino, 10129 Torino (IT)
(72) Inventor: VITALE BROVARONE, Chiara, 10141 Torino (IT); VERNE', Enrica, 10138 Torino (IT); BERGUI, Mauro, 12084 Mondovi' (Cuneo) (IT); ONIDA, Barbara, 10125 Torino (IT); BAINO, Francesco, 14100 Asti (IT); MIOLA, Marta, 10128 Torino (IT); FERRARIS, Sara, 10095 Grugliasco (Torino) (IT); TALLIA, Francesca, 13900 Biella (IT)
(74) Representative: Rambelli, Paolo
(86) International application number: PCT/IB2011/052094
(87) International publication number: WO 2011/141889

(56) References cited:
- WO-A1-02/096391
- WO-A1-2004/075989
- WO-A2-2010/023179
- US-A1- 2008 060 382
- US-A1- 2009 068 272

## Description

The present invention concerns an injectable composition for the use in bone-filling and bone-consolidation in surgery and therapy.

In particular, the invention relates to the field of injectable bone cements, for both treating of fractures caused by osteoporosis or trauma and filling gaps due, for example, to the decrease of bone mass after removal of tumors or cysts.

Furthermore, this invention may be useful to strengthen the bone structure with a view to the subsequent implant of screws or prostheses.

The request of such materials is ever growing because of the increased life expectancy.

Bone cements are generally prepared in the form of pastes that can be injected directly into the fracture site, so that, after hardening, they fill the vacuum (or consolidate the fracture) and support the surrounding bone. It is clear that such bone substitutes must provide a good short-term stability and prevent micro-movements that can delay or inhibit tissue regeneration: thus, the injected material has to harden quickly and to get fixed with the surrounding bone tissue.

The present invention is related to injectable materials that can be used in various pathological or traumatic conditions in maxillo-facial and orthopedic surgery, as well as in different applications of neurosurgery, such as vertebroplasty and kyphoplasty.

These two spinal surgical techniques are used for the treatment of osteoporotic vertebral fractures, traumatic fractures and in case of particular spinal tumors, in order to stabilize as much as possible the weakened vertebral body, to relieve the pain due to the fracture, especially when it becomes high and persistent; in addition, kyphoplasty aims also to restore the height of the vertebra to as near its pre-fracture physiological level as possible and to correct possible kyphotic deformities caused by the fracture.

These surgical approaches are increasingly frequent because, without them, the pain caused by a spinal fracture often requires a more or less prolonged bed rest. Patients in good general conditions tolerate this conservative treatment without excessive problems and, in most cases, they heal with a complete resolution of symptoms and a quick recovery of their autonomy.

Instead, bed rest represents a significant risk factor for "critical" patients, because of the possible associated severe co-morbidity: this population includes patients with limited prognosis ("very old" people, patients affected by not-controlled solid tumors), patients with a potential good prognosis after overcoming the acute phase (patients with severe liver disease awaiting transplant or in the immediate post-transplant, haematological patients receiving chemotherapy or bone marrow transplant). In these cases the percutaneous stabilization provided by vertebroplasty or kyphoplasty allows a rapid recovery of standing and walking abilities, avoiding the co-morbidity related to both the bed rest and the use of more painkillers.

These procedures usually consist in the percutaneous injection of an acrylic bone cement (based on polymethylmethacrylate - PMMA) directly into the fractured vertebral body (in case of vertebroplasty) or into a cavity created inside the vertebral body through the inflation of a balloon (in case of kyphoplasty).

After the injection, the cement hardens through polymerization and/or crosslinking processes, giving the patient an immediate sense of relief because of the decrease or even elimination of pain due to the stabilization of the vertebral body.

After injection and setting, PMMA is gradually included by a capsule of fibrous scar tissue, that makes the cement fixed with surrounding bone tissue, but it is completely inert, so it will be neither reabsorbed nor osteointegrated. Thus, the mechanical properties of the vertebral body containing PMMA are different from those of the adjacent vertebrae, resulting in possible consequences that could affect the stability of the surrounding bone (in case of vertebroplasty/kyphoplasty the risk of secondary fractures in the vertebrae adjacent to the treated one is well documented in scientific literature).

The complications are mainly related to possible cement leakage outside the vertebral body, localized temperature rise produced by hardening of acrylic cements, and, especially in case of seriously immunocompromised patients, infections.

The most desirable properties that these type of cements should possess are easy injectability, radio-opacity, mechanical properties of the hardened cement that are comparable to those of a healthy intact vertebral body.

Ideally, especially for patients with long life expectancy, the hardened cement should be gradually bioresorbed during in vivo osteointegration phenomena, with a resorption rate comparable to the kinetics of healthy bone tissue regeneration.

In fact, cements based on bioinert materials represent a permanent implant which is, therefore, barely indicated for the treatment of patients with long life expectancy.

To this end, calcium sulphate hemi-hydrate (commonly known as "Plaster of Paris"), CaSO₄·0,5H₂O, was one of the first materials investigated for bone replacements and its high biocompatibility as well as its ability to be bioresorbed, are widely documented in literature.

US Patent No. 2009/0068272 A1 describes the procedure for the synthesis of mesoporous calcium silicate powders for controlled release of bioactive agents. Paragraphs 0024 and 0031 describe particles, whose dimension ranges between 20 nm and 75 nm, obtained through acid treatment on wollastonite powders, that causes the formation of surface mesoporosity. The size of nanoparticles and the average diameter of mesopores are determined by the conditions selected for the acid treatment. Experimental verification demonstrates that the above-mentioned mesoporous calcium silicate powders can be neither mixed nor injected in combination with a proper matrix (for example, matrices in calcium sulphate hemi-hydrate or polymethylmethacrylate), resulting thus unsuitable for the preparation of injectable bone cements.

WO 2004/075989 A describes a composition used for embolization, tissue bulking or tissue augmentation, which comprises a biocompatible polymer, e.g. polymethylmethacrylate, a bio compatible solvent, a contrast agent and fumed silica.

WO 2010/023179 A2 describes an injectable bone substitute comprising a mixture of water and a powdery composition comprising a silica-based porous bioglass and calcium sulphate α-hemihydrate.

US 2008/0060382 A1 describes a biological active glass powder comprising 40-70% wt SiO₂, 5-35% wt CaO and 2-15% wt P₂O₅. The glass can have additional dopants included therein, such as SrO, Na₂O or MgO.

WO02/096391 describes a scaffold of a bioactive glass which is prepared from a foamed sol-gel material and includes an interconnected network with macropores and mesopores. Surfactants are used to obtain uniform foams.

One object of the present invention is to provide a novel injectable composition, helpful for the above-mentioned purposes, in particular a composition which would result in a rapid bone regeneration.

A further object of the invention is to provide a composition which can be used as vehicle to carry biologically active substances directly to the injection site.

For such purposes, object of the invention is an injectable composition having the characteristics defined in the claims that follow, as well as the resulting bone cement.

The composition according to the invention, when applied to an affected area of a patient, forms a composite bone cement which comprises a first phase, representing the matrix, and at least one dispersed phase.

The phase acting as the matrix may be constituted by a biocompatible powder that is able to generate, through the contact with a liquid, a solution from which one or more crystalline phases precipitate. Alternatively, the matrix may be constituted by a biocompatible powder material that, when placed in contact with a proper polymerizable liquid, is able to generate a solid material, which is mechanically resistant.

The matrix material is selected from calcium sulphate hemi-hydrate and polymethylmethacrylate; the preferred material is calcium sulphate hemi-hydrate, preferably α-calcium sulphate hemi-hydrate, which possibly may include particles of calcium sulphate di-hydrate in order to improve the injectability of the composition. Calcium sulphate hemi-hydrate is converted into calcium sulphate di-hydrate through the reaction with water, making the mixture supersaturated with respect to calcium sulphate di-hydrate, which precipitates as needle-like crystals, whose entanglement causes firstly the setting and then the hardening of the injected material.

A matrix based on calcium sulphate is completely bioresorbable in contact with body fluids in an average period of time of just over a month.

Not bioresorbable organic matrices in polymethylmethacrylate in presence of the corresponding activator agent (for example, benzoyl peroxide) are also envisaged.

The injectable composition object of the invention also includes at least a second phase that, in the resulting composite bone cement, acts as dispersed phase. This dispersed phase comprises a silica-based material, which contains other oxides, such as CaO and P₂O₅, and optionally ZrO₂ and SrO, and which is characterized by an high specific surface area, greater than 100 m²/g.

The dispersed phases are bioactive glasses with controlled mesoporosity, with a pores size variable within the range of mesopores, that is typically from 2 nm to 50 nm.

Bioactive glasses with controlled mesoporosity represent a special class of nanomaterials, i.e. materials whose properties can be tuned at the nanoscale, whose common specific feature is the presence of uniform nanopores whose size ranges within 2÷50 nm.

The synthesis of bioactive glasses with controlled mesoporosity is based on the sol-gel method, which has the advantage of being not expensive and simple and that is used to realize ceramic materials starting from liquid precursors. In the sol-gel process, which is a technique generally used for the fabrication of glass or ceramic materials, a liquid colloidal solution, commonly known as "sol", that is constituted by a suspension of solid particles (usually metal hydroxides and alkoxides or inorganic salts) into a liquid phase, gradually evolves, through hydrolysis and polycondensation reactions, towards the formation of a gel; this gel is, then, thermally treated to produce high-purity oxides. In the synthesis of bioactive glasses with controlled mesoporosity, this sol-gel method is coupled with a supermolecular self-assembly process of proper amphiphilic molecules, consisting in an organic surfactant. Specifically, the surfactant is introduced into the traditional sol-gel synthesis of bioactive glasses by its addition directly in the solution that forms the "sol", enabling to exploit the contemporary properties of hydrophilicity (tendency to dissolve in polar solvents) and hydrophobicity (tendency to dissolve in apolar environments) of such surfactant. As a result of their amphiphilic nature, surfactant molecules are able to form, through a self-assembly process, supermolecular aggregates, named micellae, whose shape and spatial arrangement can be controlled on the basis of the selection of type and concentration of surfactant.

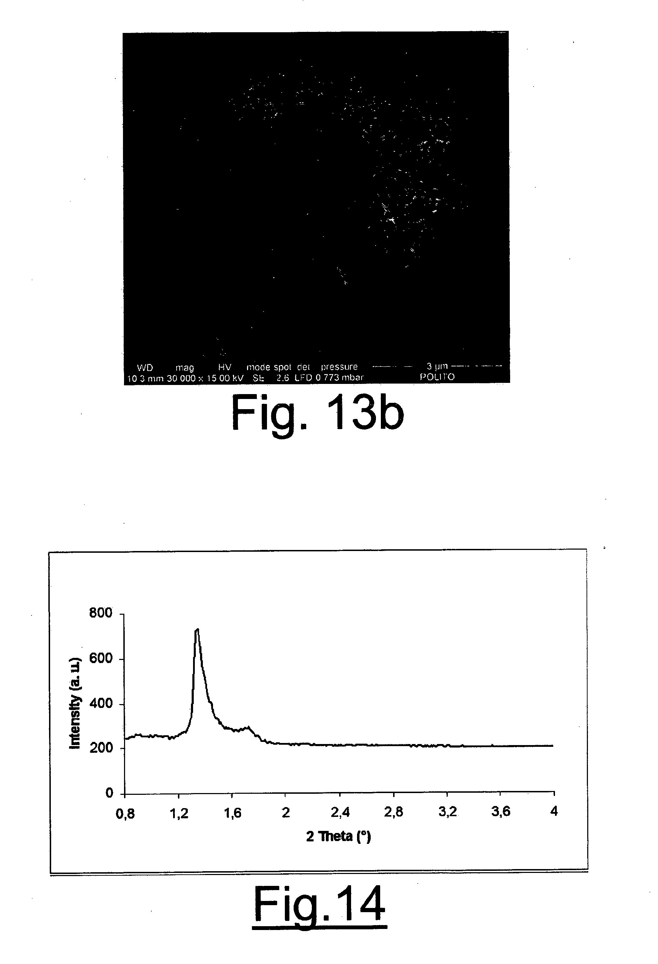

The latter, in particular, is removed through a calcination thermal treatment, leading to a bioactive glass material with controlled mesoporosity (MBG) characterized by an extremely high accessible surface area (higher than 100 m²/g and generally of the order of 300-500 m²/g). This feature, in combination with the effective control of size, distribution and order of the pores, makes it possible to act selectively controlling and tuning the properties and the behaviour to be attributed to the resulting mesoporous material, based on the type of chosen surfactant. As an example, figure 14 reports the spectrum resulted from X-ray diffraction analysis on MBG powders: diffraction peaks clearly visible within the angular range 1°-2° represent an experimental evidence of the ordered structure typical of the above-described MBGs, whose nanopores are both uniform and with controlled size.

Numerous surfactants, both ionic and non-ionic, can be used for MBG synthesis, among which the most common are for example: block copolymers of poly(ethylene glycol)-poly(propylene glycol)-poly(ethylene glycol) (e.g. Pluronic P123 and F127), cetyltrimethylammonium bromide (CTMABr), isononylphenoxy-poly(ethylene) oxide (NONFIX 10).

Therefore, the presence of the nanoporous structure inside a bioactive glass increases its exposed surface area, accelerating the complex mechanism of ion exchange with body fluids, which is the base of the bioactivity of these materials (e.g. their ability to establish a strong chemical bond with surrounding bone tissue through the rapid formation of sub-micrometrical hydroxyapatite crystals on their surface). Hence, the in vivo implant of a MBG is potentially able to stimulate a rapid bone regeneration, enabling a more effective and active patient rehabilitation, which represents a very important feature in case of osteoporotic patients.

Moreover, a further advantage consists in the possibility of using MBG nanopores as vehicles for the transport and the controlled release of drugs or other biomolecules in order to relieve pain of patients, reduce inflammation or treat specific diseases. In particular, specific growth factors or anti-tumoral drugs can be transported.

MBGs, according to the present invention, include bioactive glasses with the following composition:
SiO₂: from 60 to 85% by moles;
CaO: from 10 to 30% by moles;
P₂O₅: from 2 to 10% by moles.
and optionally other oxides, such as ZrO₂ and/or SrO, from 2% to 15% referred to 100% by moles of the 3 above-mentioned oxides.

Preferred specific surface area: 300-500 m²/g (data calculated from nitrogen sorption curve through BET analysis)

Preferred/preferable pores size: 2-10 nm.

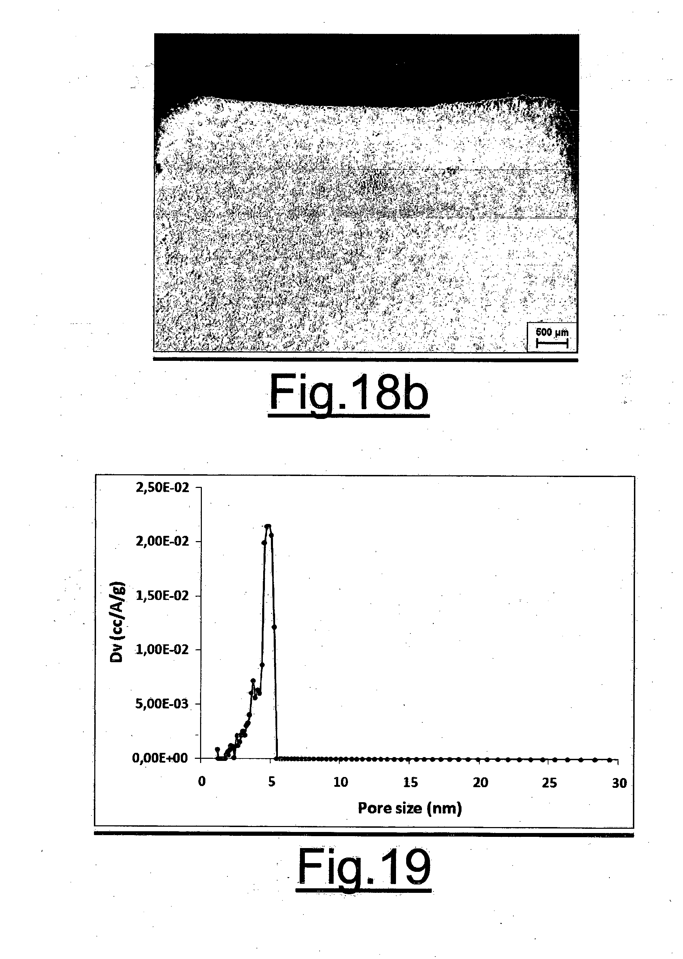

According to a preferred but not binding embodiment, MBG, to which examples from 1 to 7 refer, exhibits a unimodal distribution of pores diameters characterized by a maximum peak value of pores size around 4,7 nm, as shown in figure 19.

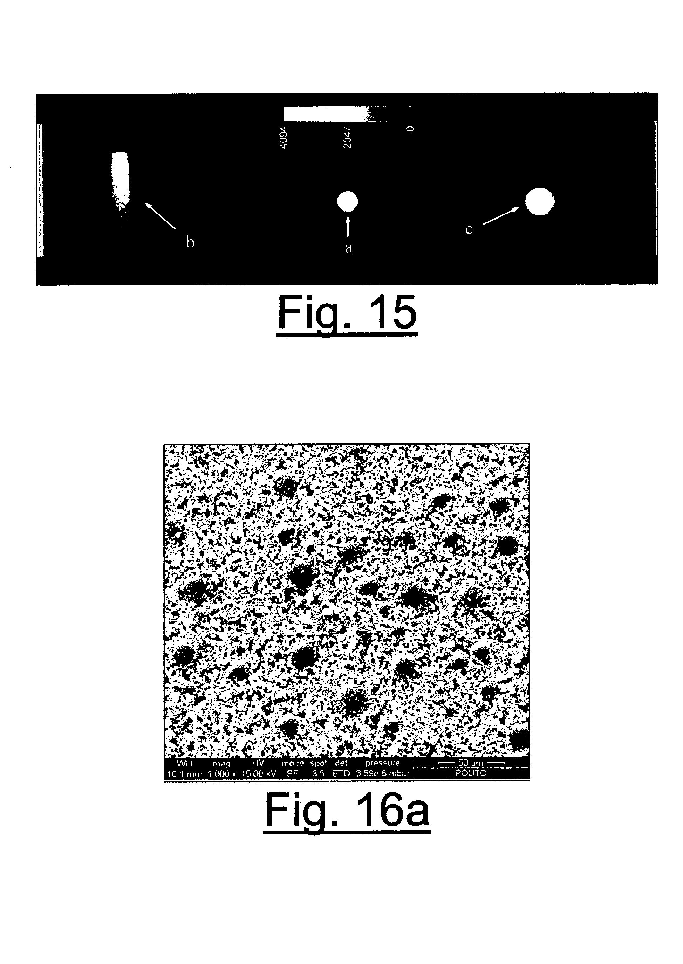

An essential requirement for vertebral cements consists in the need to have a suitable radio-opacity degree to ensure its visualization under radioscopic guidance, both during the positioning inside the vertebral body in the percutaneous injection procedure (in order to observe any possible leakage) and during the subsequent follow-up checks on the patient. For this reason, in order to impart radio-opacity to the injectable composite material, radio-opaque powders, such as BaSO₄, are usually added. In addition, radio-opacity can be achieved using dispersed phases which contain also ZrO₂ and/or SrO. In a preferred and innovative embodiment, the invention allows the use of bioactive mesoporous glasses with controlled mesoporosity (MBG) within the range 2 - 50 nm, preferably from 2 nm to 10 nm, containing an inherently radio-opaque oxide (such as ZrO₂ or SrO), named hereafter MBGZ; in this embodiment the invention provides composite cements that combine bioactive properties and radio-opacity in only one dispersed phase, thus avoiding the need of adding a radio-opaque agent as described above: in fact, the added radio-opaque agent would represent a further dispersed phase which would cause the worsening of miscibility, injectability and mechanical properties, especially bending strength and dynamic fatigue performance, of the composite cement. In particular, as described in detail in examples 8 and 9 reported below, the feasibility of both MBGZ synthesis and introduction of MBGZ particles as dispersed phase within a calcium sulphate matrix has been demonstrated: as shown in figure 15, both MBGZ powder and the resulting composite cement are considerably radio-opaque. This fact represents a great innovation of this invention because no publications regarding MBGZ were detected in scientific literature or in patent databases and there is not any composite cement for vertebroplasty that does not contain BaSO₄ or other radio-opaque additives currently available on the market.

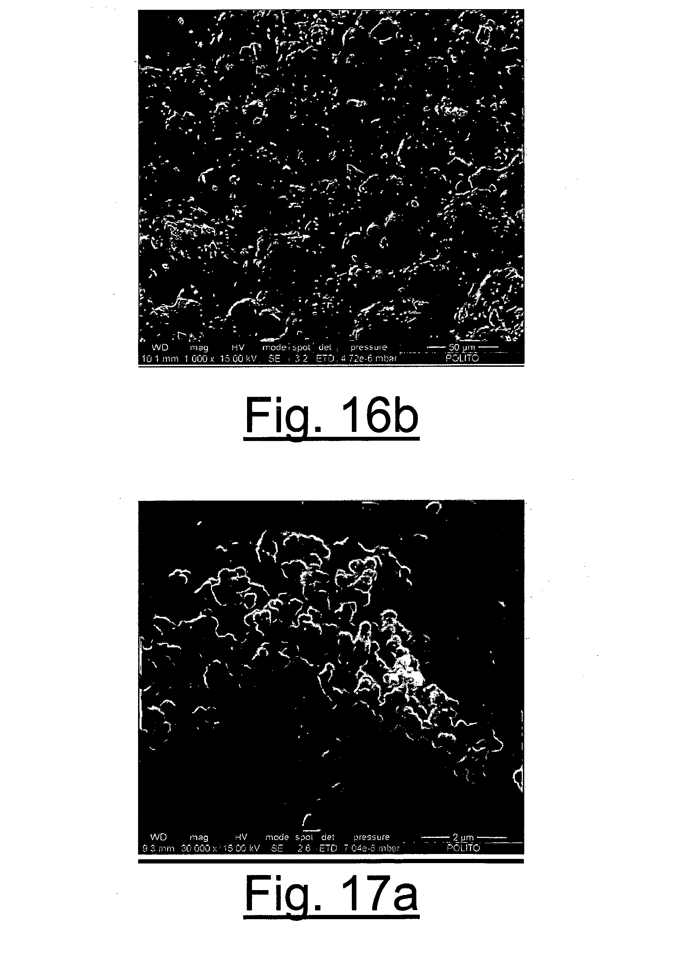

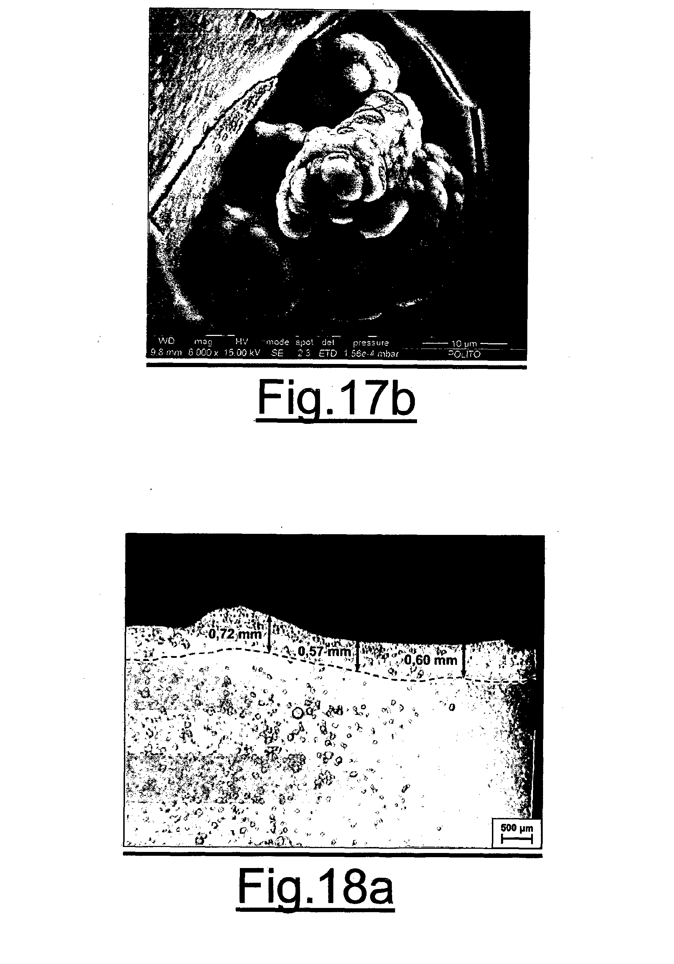

The above-mentioned MBGs are characterized by an osteoinductive behaviour, that consists in an extremely high ability to stimulate the nucleation of sub-micronic crystals of hydroxyapatite on their surface. To this end, preferred size of MBG particles ranges between 5 µm and 32 µm (minimum dimension resulting from sieving). In fact, unexpected experimental evidences have demonstrated that the utilization of a powder granulometry below 32 µm maximizes the exposure of MBG particles on the surface of the resulting hardened cement, since larger particles have difficulty to emerge from the matrix within they are trapped (this effect is clearly visible comparing figures 16a and 16b). MBG exposure is extremely important, because, with a view to the application of the injectable compositions object of the present invention as bone cements, particles exposed on the surface are those which enable the bioactive and osteoinductive effect after the contact with body fluids, feature that is the main purpose of their use as dispersed phase. In this regard, capillarity tests were carried out to evaluate the capillary absorption ability of fluids from hardened composite cements with polymethylmethacrylate matrix, by varying the dispersed phase: in particular, composite cements prepared according to example 6 were compared to cements characterized by the same matrix and the same composition but containing a different dispersed phase, since the MBG powder was substituted by particles of a traditional not-mesoporous bioactive glass with the same size (sieved below 32 µm). Capillarity tests were carried out by partially soaking each sample in a mixture of simulated body fluid (Kokubo's SBF (Kokubo, Takadama - Biomaterials 27 (2006) 2907-2915)) and a coloured liquid, which acts as contrast agent that enables to detect the depth of capillary absorbed fluid from the contact surface. As shown by the observations of the sections of such samples through optical microscope (figures 18a and 18b), a considerable absorption was assessed for the composite containing MBG, since a capillary depth of 600 µm was measured for this sample, whereas no absorption layer was noticed in the composite containing the traditional not-mesoporous bioactive glass. This outcome represents a further unexpected demonstration of the greater technical effect caused by the utilization of MBG with optimized size because particles sieved below 32 µm are highly exposed on the surface and, when the hardened cement is placed in contact with body fluids, their nanopores determine a remarkable capillary absorption that supports and promotes their osteoinductive function, resulting in a considerable increase of MBG bioactivity in respect to a not mesoporosus bioactive glass. Moreover, particles with size lower than 5 µm show a high tendency to agglomerate: this effect irreparably affects the miscibility between solid and liquid phases and the consequent injectability of the resulting paste, preventing therefore to satisfy the viscosity requirements needed for an injectable bone cement.

These glasses may be used as a vehicle for the transport of biologically active substances, including drugs, such as antibiotics, anti-inflammatories, painkillers and anti-tumoral agents, as well as growth factors, such as for example morphogenetic proteins.

In one embodiment, a further phase may be added to the matrix material of the injectable composition, especially in case of bioresorbable injectable matrices: this further material acts as a dispersed phase in the hardened composition, in order to impart better mechanical properties and to provide support and stabilization to the re-growing physiological tissue.

This dispersed phase can be in the form of bioresorbable glass powders or fibres, preferably non-porous, having the following composition:
P₂O₅: from 40 to 60% by moles
CaO: from 10 to 40% by moles (preferably between 20 and 30% by moles)
and optionally containing other metal oxides, such as Na₂O, MgO, K₂O and TiO₂, as a complement to 100% by moles.

The further addition of the above-mentioned bioresorbable dispersed phase is useful to induce, during the osteointegration of the injected material, the formation of a porous network that may be helpful for the transport of nutrients and waste products and can also promote the bone regeneration process.

In order to considerably increase the mechanical properties, especially in case of bioresorbable matrices, another glass material can be introduced as a further dispersed phase in the form of powders or fibres, preferably non-porous, whose size ranges preferably within 5 and 100 µm, having the following composition:
SiO₂: from 40 to 65% by moles
CaO: from 10 to 35% by moles;
and optionally containing other oxides, such as Na₂O, MgO, K₂O, Al₂O₃, ZrO₂ and TiO₂ as a complement to 100% by moles. This dispersed phase can be thermal treated before its addition to the powders, in order to induce a partial devetrification of the glass, resulting in the nucleation of one or more crystalline phases that increase its mechanical strength (glass-ceramic).

In compositions based on a bioresorbable matrix, such as for example calcium sulphate hemi-hydrate, the addition of one or more dispersed phases has also the advantage of reducing the resorption rate of the matrix, making it more compatible with the kinetics of bone tissue regeneration. For example, tests on different cements, composed of simple calcium sulphate di-hydrate or composite materials made by the combination of a calcium sulphate di-hydrate matrix and one or more dispersed phases, were carried out in vitro by soaking the samples in an acellular simulated body fluid that aims to mimic the ion concentration of human plasma (Kokubo's SBF), obtaining the following results:
- a cement constituted only by calcium sulphate di-hydrate matrix shows a weight loss of about 25% by weight after 1 week of soaking in SBF;
- a composite material, prepared in accordance with example 1 that follows, shows a weight loss of about 8% by weight after 7 days of soaking in SBF;
- a composite material, prepared in accordance with example 2 that follows, shows a weight loss of about 7% by weight after 7 days of soaking in SBF;
- a composite material, prepared in accordance with example 3 that follows, shows a weight loss of about 5% by weight after 7 days of soaking in SBF.

The percentage of the matrix component is typically from 40% to 95% by weight, referred to the 100% by weight of total amount of matrix and dispersed phase/phases. In particular, the percentage of the matrix material ranges preferably between 60% and 90% by weight.

In case of inorganic matrices for the injectable composition object of the invention, the dry powder, constituted by the matrix and the dispersed phases, is mixed with an aqueous liquid, which also aims to ensure the injectability of the composite paste. This liquid phase can be enriched with a radio-opaque agent and optionally with an oily component that facilitates the injection of the composition.

Typically, the aqueous phase is distilled water or a balanced saline solution; the amount of the aqueous liquid mixed with the powders typically ranges from 0,10 to 1 ml per gram of the total dry powder and preferably between 0,45. and 0,60 ml/g.

In case of organic matrices for the injectable composition object of the invention, the dry powder, constituted by the matrix and the dispersed phases, is mixed with a liquid mixture of monomer, catalyst and inhibitor, that aim at ensuring the injectability and the subsequent hardening of the composite. For example, when a PMMA matrix is used, the monomer is typically MMA (methyl-methacrylate), the catalyst may be dimethyl toluidine, the inhibitor may be hydroquinone.

In this case, the volume of the liquid phase mixed with the powders ranges from 0,20 to 0,80 ml per gram of the total dry powder and preferably between 0,30 and 0,60 ml/g.

Whatever is the material used as matrix, the liquid phase can be enriched with a radio-opaque agent and optionally with an oily component that facilitates the injection of the composition.

The viscosity of the injectable material should be adapted to make it injectable into the bone for 1-10 minutes after the beginning of the mixing procedure of the different components. Moreover, the specific formulation of both the composition and the selected liquid phase should be tuned in order to obtain preferably a setting time that varies between 10 and 30 minutes from the dough injection. To achieve the injectability and the setting times described above, a retardant agent, such as for instance a carboxylic acid (e.g. citric acid, succinic acid, malic acid, tartaric acid), can be added either to the powder resulting from the mixture of matrix and dispersed phases (which represents the composition object of the invention) or to the liquid needed to provide the injectability.

The composition can be marketed in the form of a kit that contains measured amounts of each component - matrix material, dispersed phases, liquid phase - as well as all the accessories that the user needs for the preparation and the injection. Further features and advantages of the composition according to the invention will be apparent from the following examples, that, however, should not be construed as limiting the invention in any way.

In the appended drawings:

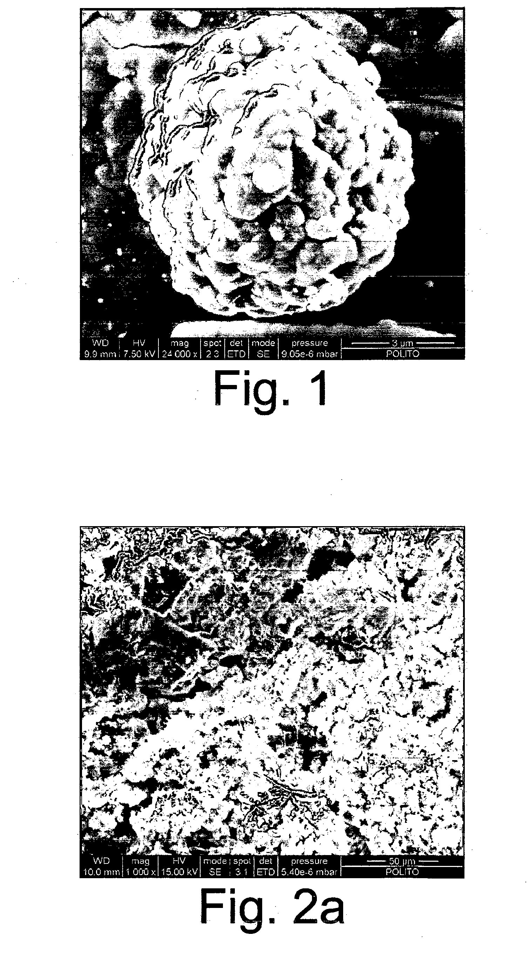

- figure 1 illustrates a micrograph of an hydroxyapatite layer precipitated on MBG powders after 24 hours of soaking in SBF;
- figure 2a illustrates a micrograph of the composite material, prepared according to example 1 that follows, constituted by a calcium sulphate hemi-hydrate matrix containing MBG, after 8 hours of soaking in SBF: small agglomerates of hydroxyapatite are visible on the surface of the composite material;

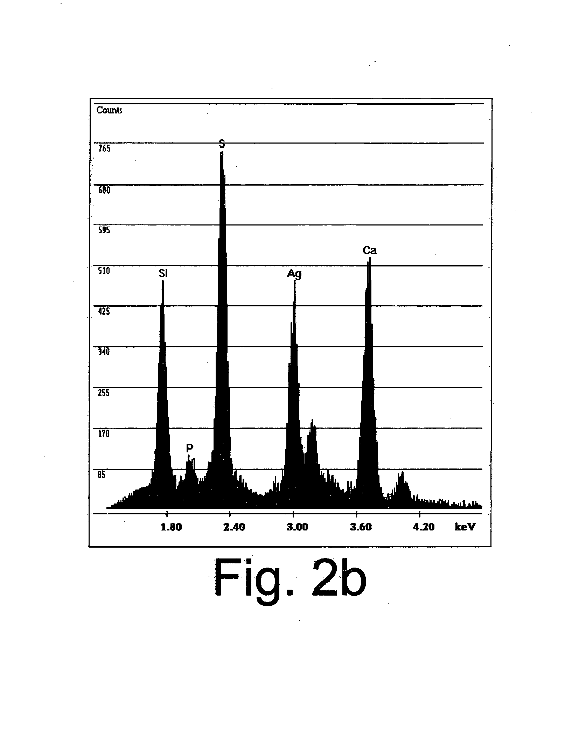

- figure 2b reports an EDS composition analysis of the whole area exhibited in figure 2a: the visible peaks correspond to the constitutive elements of the composite and show an increase in the amounts of Ca and P if compared with those of the starting composition of the dispersed MBG particles, suggesting the precipitation of hydroxyapatite crystals, that demonstrates the extremely high bioactivity of such material;

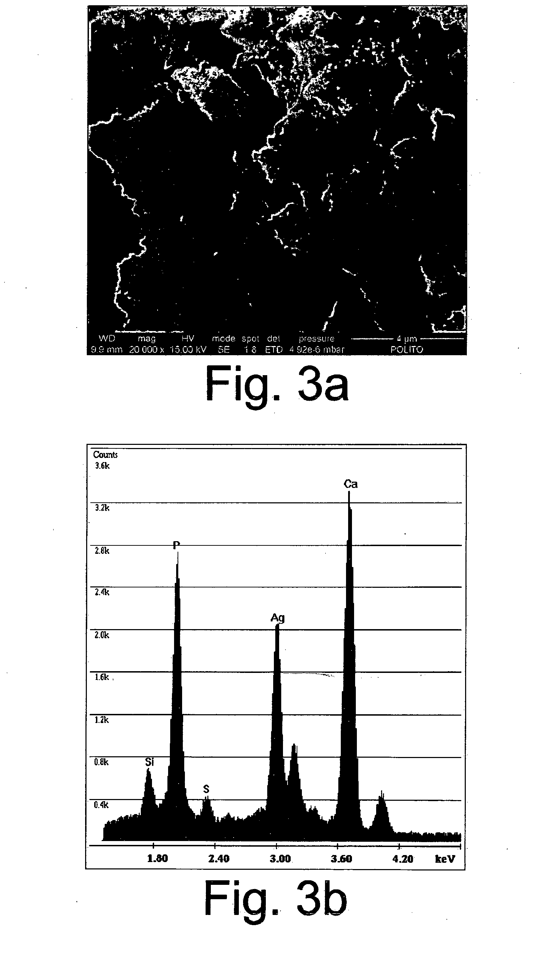

- figure 3a illustrates a high-magnification micrograph of some agglomerates of hydroxyapatite particles nucleated on the composite prepared according-to example 1 after 1 day of soaking in SBF;
- figure 3b reports an EDS composition analysis of the material surface visible in figure 3a, in which the peaks corresponding to Ca and P stand out clearly, confirming that the crystals are actually hydroxyapatite;

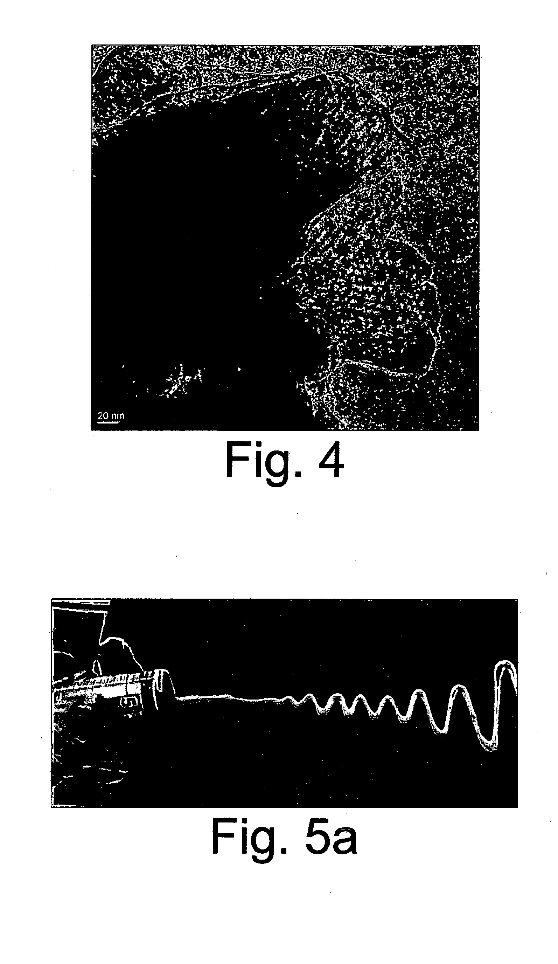

- figure 4 illustrates a TEM image of the MBG used in all examples from 1 to 7 described below, that shows an ordered and regular nanoporosity;
- figure 5a is a macrograph that illustrates the injectability of the composite material prepared in accordance with examples 2-3 and 4;

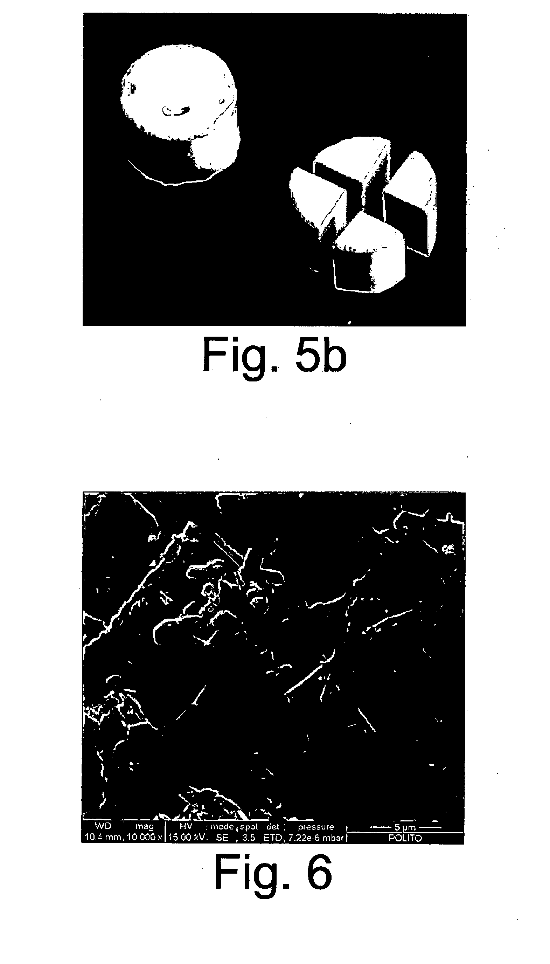

- figure 5b illustrates a macrograph that shows an example of cylindrical sample (diameter = 16 mm; height = 10 mm) of the composite material obtained according to examples 2-3 and 4, both entire and in section;
- figure 6 illustrates a micrograph of the surface of the composite material prepared in accordance with example 2, that clearly exhibits calcium sulphate di-hydrate crystals (precipitated after mixing the calcium sulphate hemi-hydrate powder with the liquid phase) among which the particles of the two dispersed phases (MBG and a SiO₂-CaO-Na₂O-Al₂O₃ based glass) are trapped and uniformly distributed;

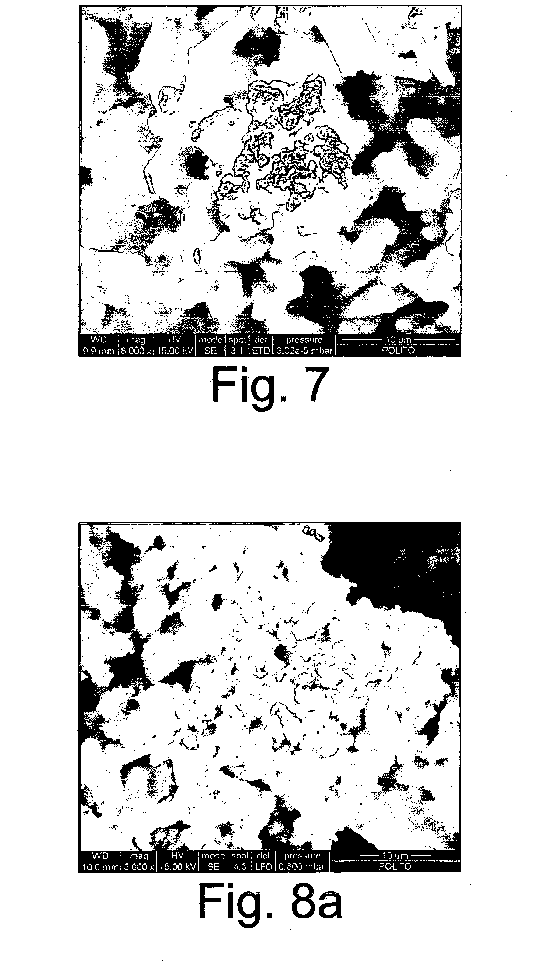

- figure 7 illustrates a micrograph of the surface of the composite material realized according to example 2 after 1 week of soaking in SBF. In particular, it shows the magnification of some agglomerates of hydroxyapatite particles nucleated on the composite surface: it is important to underline that, despite the excellent bioactive properties of the MBG used as dispersed phase in the composite, the material is not covered by a uniform hydroxyapatite (HAp) layer, because the HAp precipitates are bioeroded with the matrix;
- figure 8a illustrates a micrograph that shows a magnification of the residual powder filtered from the SBF in which the sample observed in figure 7 was soaked: consistently with what is described above, it clearly exhibits a large amount of agglomerates of hydroxyapatite particles detached from the sample surface because of the resorption;

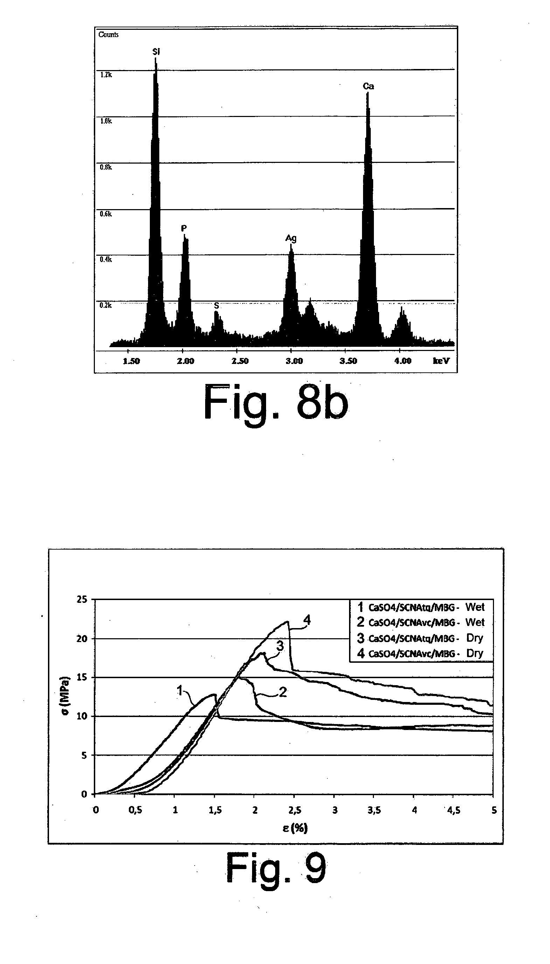

- figure 8b reports an EDS composition analysis of a limited area of the material observed in figure 8a, in which the height of the peaks corresponding to Ca and P confirms that the micro-crystals are actually hydroxyapatite precipitated on MBG particles, whose presence is proven by the peak of Si; in addition, the peak of S denotes the presence of calcium sulphate di-hydrate;
- figure 9 is a diagram that illustrates the comparison between the σ-ε curves resulted from the compressive strength testing of the two different composite materials prepared by mixing the calcium sulphate hemi-hydrate powder with MBG particles and a second dispersed phase, in accordance with example 2 (reported as "CaSO₄/SCNAtq/MBG", in which the second phase is a completely amorphous glass) and example 3 (reported as "CaSO₄/SCNAvc/MBG", in which the second phase is a glass-ceramic material). Both composites were tested in "wet" conditions (after 24 hours after the preparation), which simulate the in vivo contact with body fluids, and in "dry" conditions (after 1 week after the preparation), leading to the following results in terms of compressive strength:
   CaSO₄/SCNAtq/MBG - Wet: 12,7 MPa;
   CaSO₄/SCNAvc/MBG - Wet: 15,1 MPa;
   CaSO₄/SCNAtq/MBG - Dry: 17,0 MPa;
   CaSO₄/SCNAvc/MBG-Dry: 22,1 MPa.

Consistently with the theoretical expectations, the presence of the second dispersed phase in form of a glass-ceramic material provides an increase in the mechanical properties of the composite in which it is introduced, if compared with the same material characterized by the second dispersed phase in form of a completely amorphous glass. Moreover, an increase in both the strength and the stiffness of the above-mentioned composite materials is observed after the complete evaporation of the excess of water, needed to ensure their injectability;

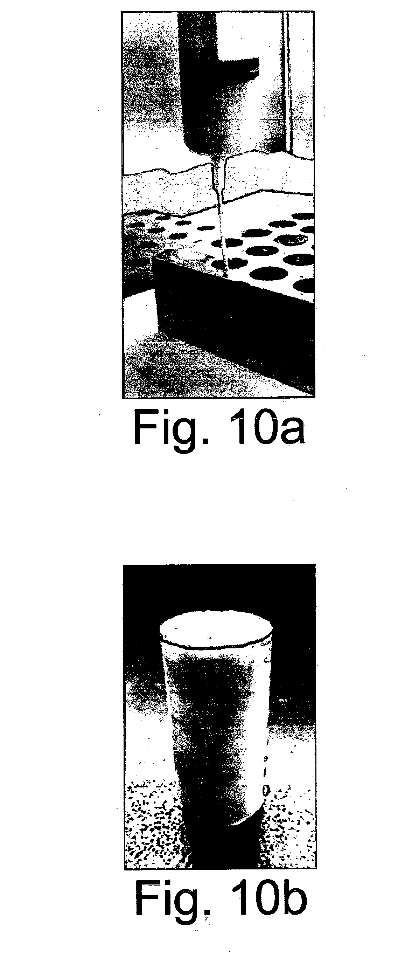

- figure 10a illustrates a macrograph that demonstrates the injectability of the composite material prepared in accordance with examples 5-6 and 7;
- figure 10b illustrates a macrograph that shows an example of cylindrical sample (diameter = 12 mm; height = 24 mm) of the composite material obtained according to examples 5-6 and 7;

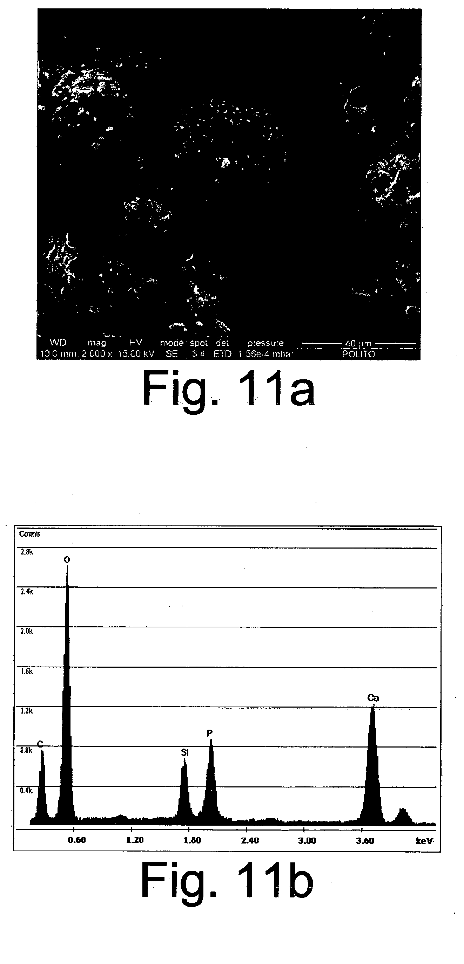

- figure 11a illustrates a micrograph of the surface of the composite material realized according to example 5 after 1 day of soaking in SBF, in which the formation of many agglomerates of hydroxyapatite micro-crystals is clearly visible both on MBG particles that emerge from the matrix and on the matrix itself;
- figure 11b reports an EDS composition analysis of the material observed in figure 11a;

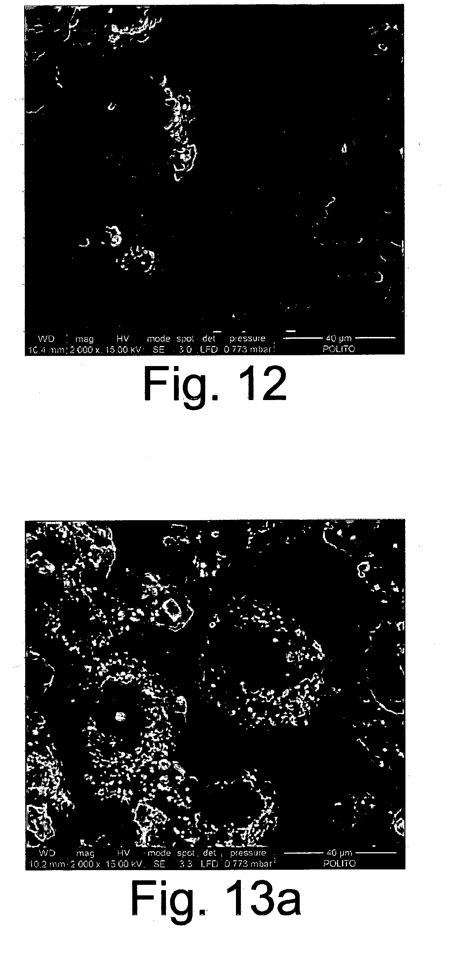

- figure 12 illustrates a micrograph of the surface of the composite material realized according to example 5 after 3 days of soaking in SBF, which shows an increased amount of hydroxyapatite micro-crystalline agglomerates than that observed in figure 11a, consistently with the longer duration of the immersion;
- figure 13a illustrates a micrograph of the surface of the composite material realized according to example 5 after 7 days of soaking in SBF: as a consequence of the further increase of the test duration compared with the two previous figures, the micro-crystalline agglomerates of hydroxyapatite are homogeneously distributed, so that they form a layer that completely covers both the polymeric matrix and the emerging MBG particles;
- figure 13b illustrates a micrograph that shows a magnification of the sub-micrometrical hydroxyapatite crystals reported in figure 13a, which cover the whole exposed surface of the composite prepared as described in example 5 after 7 days of soaking in SBF;
- figure 14 reports the spectrum resulted from X-ray diffraction analysis on MBG powders in the range of 2θ angle between 0,6° and 5°: diffraction peaks clearly visible within the angular range 1°-2° demonstrate that nanoporous particles possess a regular structure. For the specific example under consideration, the selected surfactant is Pluronic P123, which determines nanopores characterized by an hexagonal shape with an average pores size of 4,7 nm, as seen in figure 19;
- figure 15 illustrates an X-ray image, obtained through the radiological equipment specific for vertebroplasty, that allows to compare the radio-opacity level of: (a) commercial vertebral cement (CementoFixx® produced by Optimed), that represents the reference material to evaluate the radio-opacity; (b) MBGZ powder, synthesized according to the following example 8; (c) composite cement prepared as described in example 9 below, that is characterized by a calcium sulphate matrix containing MBGZ as one of the dispersed phases. From this figure it is clear that the radio-opacity level conferred by the MBGZ, both as in the form of powder and as dispersed phase in an injectable cement, can be considered more than satisfactory because it is totally comparable, or even higher, to that of vertebral cements available now on the market;
- figure 16a illustrates a micrograph of the surface of the composite material realized according to example 6 using MBG particles smaller than 32 µm, while figure 16b illustrates a micrograph, at the same magnification of figure 16a, of the composite material realized according to example 6 using MBG particles whose size ranges within 75 and 106 µm: in both figures polymethylmethacrylate particles, which are spherical, and MBG ones, which are sharp and irregular, are observed. Comparing the two images, it is clearly evident that smaller the size of the particles of glass dispersed phase greater their exposure on the surface: in fact, whereas figure 16b shows a clear prevalence of spherical matrix particles among which only a very limited number of MBG particles emerges with a uniform distribution, figure 16a exhibits a reversal situation, characterized by an homogeneous layer of dispersed phase among which only few polymethylmethacrylate particles are able to come out. Such unexpected outcomes underline the importance of making an accurate modulation of the particles size and selecting the range that maximizes the exposure of dispersed phase on the surface, in order to optimize its bioactive and osteoinductive function when in contact with body fluids;
- figure 17a illustrates a micrograph of a globular hydroxyapatite agglomerate nucleated on MBGZ (prepared in accordance with example 8) after 72 hours of soaking in SBF, demonstrating the excellent bioactivity of MBGZ;
- figure 17b illustrates a micrograph which shows a layer of hydroxyapatite micro-crystals precipitated on MBGZ (prepared in accordance with example 8) after 7 days of soaking in SBF, that represents a further proof of the extremely high bioactivity provided by MBGZ;
- figure 18a is an image obtained through optical microscope which illustrates the section of a hardened sample of a cement prepared in accordance with example 6, that is a composite constituted by a polymethylmethacrylate matrix containing 20% by weight of MBG powder smaller than 32 µm, as resulted from capillarity test: this was carried out by partially soaking the sample (for half of its height) in a mixture of SBF and a red liquid for a fixed time. From this figure a depth of capillary absorption of about 600 µm is clearly noticeable: this fact demonstrates that, when MBG particles are highly exposed on the surface, as previously shown by figure 16a, the presence of their nanopores enables a remarkable capillary absorption of fluids, which is an effect that promotes and increases the bioactivity provided by the osteoinductive behaviour of MBG;
- figure 18b is an image obtained through optical microscope which illustrates the section of a hardened sample of a composite cement constituted by a polymethylmethacrylate matrix containing 20% by weight of powder smaller than 32 µm of a traditional not-mesoporous bioactive glass (prepared through melting, quenching, milling and sieving), as resulted from capillarity test: this was carried out by partially soaking the sample (for half of its height) in a mixture of SBF and a red liquid for a fixed time. From this figure no layer of fluid absorption can be noticed: therefore, in absence of nanopores almost no capillarity is observed and, by comparison with figure 18a, this outcome further confirms the greater technical effect provided by the utilization of MBG with optimized size rather than a traditional bioactive glass;
- figure 19 reports a curve that describes the size distribution of the nanopores of MBG (used in all examples from 1 to 7), obtained by nitrogen adsorption experiment, calculated using DFT method.

### Example 1

Composition containing:
- Matrix material
   α-calcium sulphate hemi-hydrate: 80% by weight
- Dispersed phase
   MBG: 20% by weight
- MBG composition:
   SiO₂:80% by moles
   CaO: 15% by moles
   P₂O₅: 5% by moles
   Particle size: below 20 µm
   Specific surface (BET): 307 m²/g
   Average pore size: 4,7 nm
   (calculated using DFT method)

The bone cement composition was obtained by adding to the mixture of the above-mentioned powders an amount of water ranging between 0,1 and 1 ml per gram of the total dry powder and preferably between 0,5 and 0,6 ml/g.

### Example 2

Composition containing:
- Matrix material
   α-calcium sulphate hemi-hydrate: 70% by weight
- First dispersed phase
   MBG: 10% by weight
- MBG composition:
   SiO₂: 80% by moles
   CaO: 15% by moles
   P₂O₅: 5% by moles
   Particle size: below 20 µm
   Specific surface (BET): 307 m²/g
   Average pore size: 4,7 nm
   (calculated using DFT method)
- Second dispersed phase
   SiO₂-CaO-Na₂O-Al₂O₃ based glass: 20% by weight
- Composition of the second dispersed phase:
   SiO₂: 57% by moles
   CaO: 34% by moles
   Na₂O: 6% by moles
   Al₂O₃:3% by moles
   Particle size: below 20 µm
   Specific surface: a few m²/g
   Almost zero porosity
   Liquid to powder ratio ranges between 0,5 ml/g and 0,6 ml/g.

### Example 3

Composition containing:
- Matrix material
   α-calcium sulphate hemi-hydrate: 70% by weight
- First dispersed phase
   MBG: 10% by weight
- MBG composition:
   SiO₂:80% by moles
   CaO: 15% by moles
   P₂O₅: 5% by moles
   Particle size: below 20 µm
   Specific surface (BET): 307 m²/g
   Average pore size: 4,7 nm
   (calculated using DFT method)
- Second dispersed phase
   20% by weight of SiO₂-CaO-Na₂O-Al₂O₃ based glass-ceramic containing crystals of β-wollastonite Ca(SiO₃):

- Composition of the second dispersed phase:
   SiO₂: 57% by moles
   CaO: 34% by moles
   Na₂O: 6% by moles
   Al₂O₃:3% by moles
   Particle size: below 20 µm
   Specific surface: a few m²/g
   Almost zero porosity
   Liquid to powder ratio ranges between 0,5 ml/g and 0,6 ml/g.

### Example 4

Composition containing:
- Matrix material
   α-calcium sulphate hemi-hydrate: 60% by weight
- First dispersed phase
   MBG: 10% by weight
- MBG composition:
   SiO₂: 80% by moles
   CaO: 15% by moles
   P₂O₅: 5% by moles
   Particle size: below 20 µm
   Specific surface (BET): 307 m²/g
   Average pore size: 4,7 nm
   (calculated using DFT method)
- Second dispersed phase
   30% by weight of SiO₂-CaO-Na₂O-Al₂O₃ based glass-ceramic containing crystals of β-wollastonite Ca(SiO₃):
- Composition of the second dispersed phase:
   SiO₂: 57% by moles
   CaO: 34% by moles
   Na₂O: 6% by moles
   Al₂O₃: 3% by moles
   Particle size: below 20 µm
   Specific surface: a few m²/g
   Almost zero porosity
   Liquid to powder ratio ranges between 0,5 ml/g and 0,6 ml/g.

### Example 5

Composition containing:
- Matrix material
   polymethylmethacrylate: 70% by weight
- Dispersed phase
   MBG: 30% by weight
   MBG composition:
   SiO₂: 80% by moles
   CaO: 15% by moles
   P₂O₅: 5% by moles
   Particle size: below 20 µm
   Specific surface (BET): 307 m²/g
   Average pore size: 4,7 nm
   (calculated using DFT method)
      - overall ratio: liquid phase (monomer, catalysts and inhibitors) / solid phase (polymethylmethacrylate, initiator and MBG) = 0,5 ml/g
      - ratio: liquid phase (monomer, catalysts and inhibitors) / (polymethylmethacrylate and initiator) = 0,7 ml/g.

### Example 6

Composition containing:
- Matrix material
   polymethylmethacrylate: 80% by weight
- Dispersed phase
   MBG: 20% by weight
   MBG composition:
   SkiO₂: 80% by moles
   CaO: 15% by moles
   P₂O₅: 5% by moles
   Particle size: below 32 µm or in the range 75-106 µm
   Specific surface (BET): 307 m²/g
   Average pore size: 4,7 nm
      (calculated using DFT method)
- overall ratio: liquid phase (monomer, catalysts and inhibitors) / solid phase (polymethylmethacrylate, initiator and MBG) = 0,5 ml/g
- ratio: liquid phase (monomer, catalysts and inhibitors) / (polymethylmethacrylate and initiator) = 0,6 ml/g.

Mechanical compressive tests according to ISO 5833-2002 standard (that is specific for acrylic resin cements for biomedical applications) were performed on the two compositions object of the present example (among which the only difference is the granulometry of powders of the dispersed phase and which are identified respectively as "PMMA/MBG<32µm - 80/20%wt" and "PMMA/MBG [75;106]µm - 80/20%wt") in order to determine the values of their compressive strength. For this test, the selected reference is a cement composed of only polymethylmethacrylate matrix (identified as "PMMA - 100%wt"), whose samples were tested in the same experimental conditions. The values of compressive strength for the tested cements are reported in the following table in the form required by the standard, that is the strength value in correspondence to both the 2% offset and the upper yield point:

| **Tested cement** | **R at ε = 2% [MPa]** | **R at upper yield point [MPa]** |
|---|---|---|
| PMMA 100%wt | 88,4 ± 5,3 | 90,1 ± 5,6 |
| PMMA/MBG<32µm - 80/20%wt | 97,7 ± 7,5 | 98,1 ± 7,4 |
| PMMA/MBG[75;106]µm - 80/20%wt | 94,2 ± 2,4 | 94,4 ± 2,4 |

These results suggest that the addition of MBG particles, besides imparting bioactivity to the composite cement, significantly improves its mechanical compressive strength if compared to the reference cement composed of matrix only. Such increase is much more noticeable when smaller dispersed particles are used because they ensure better mixing and dispersion of the different phases, thus providing a greater homogeneity in the properties of the resulting composite material. This observations, besides being a considerable advantage shown by the compositions object of the present invention, represents an unexpected outcome with respect to what is reported in some publications present in the scientific literature, which describe a decrease of the compressive strength when a dispersed phase is added to composite cements compared with simple polymethylmethacrylate, for instance: Rentería-Zamarrón D., Cortés-Hernández D.A., Bretado-Aragón L., Ortega-Lara W., "Mechanical properties and apatite-forming ability of PMMA bone cements", Materials and Design, 2009, vol. 30, pag. 3318-3324.

### Example 7

Composition containing:
- Matrix material
   polymethylmethacrylate: 90% by weight
- Dispersed phase
   MBG: 10% by weight
   MBG composition:
   SiO₂:80% by moles
   CaO: 15% by moles
   P₂O₅: 5% by moles
   Particle size: below 20 µm
   Specific surface (BET): 307 m²/g
   Average pore size: 4,7 nm
   (calculated using DFT method)
- overall ratio: liquid phase (monomer, catalysts and inhibitors) / solid phase (polymethylmethacrylate, initiator and MBG) = 0,4 ml/g
- ratio: liquid phase (monomer, catalysts and inhibitors) / (polymethylmethacrylate and initiator) = 0,5 ml/g.

### Example 8

Here is described an example of synthesis of a bioactive mesoporous glass containing zirconia as radio-opaque agent, identified as MBGZ, having the following composition: SiO₂: 73% by moles
CaO: 15% by moles
P₂O₅: 5% by moles
ZrO₂: 7% by moles

MBGZ was synthesized by using the commercial non-ionic block copolymer Pluronic P123 (EO₂₀PO₇₀EO₂₀, where "EO" is poly(ethylene glycol) and "PO" is poly(propylene glycol)) as organic surfactant, which acts as structure-directing agent for pores formation.

In a typical synthesis of MBGZ, a synthesis batch is prepared by dissolving P123 (4 g), tetraethylorthosilicate (TEOS, 6,10 g), calcium nitrate tetrahydrate (Ca(NO₃)₂·4H₂O, 1,42 g), triethylphosphate (TEP, 0,73 g), zirconium propoxide (0,92 g), acetylacetone (Acac, which acts as a stabilizer to prevent the zirconium propoxide from uncontrollable hydrolysis and consequent precipitation, 0,10 g) and HCl 0,5 M (1 g) in ethanol (60 g). This synthesis batch is continuously stirred at 35°C for 24 hours. The resulting sol is cast into Petri dishes to undergo an ageing phase (24 hours at room temperature and 24 hours at 120°C), during which the evaporation-induced self-assembly (EISA) process occurs. The dried gel is, finally, calcined at 750°C for 5 hours to obtain the final MBGZ product.

In order to obtain MBGZ powder to be used as dispersed phase in the composite cements object of the present invention, MBGZ is ground and sieved to select particles of the desired size.

### Example 9

Composition containing:
- Matrix material
   α-calcium sulphate hemi-hydrate: 70% by weight
- First dispersed phase
   MBGZ: 10% by weight
- MBGZ composition:
   SiO₂: 73% by moles
   CaO: 15% by moles
   P₂O₅: 5% by moles
   ZrO₂: 7% by moles
   synthesized as described in example 8.
   Particle size: below 20 µm
   Specific surface (BET): 320 m²/g
   Average pore size: 4,4 nm
   (calculated using DFT method)
- Second dispersed phase
   20% by weight of SiO₂-CaO-Na₂O-Al₂O₃ based glass-ceramic containing crystals of β-wollastonite Ca(SiO₃):
- Composition of the second dispersed phase:
   SiO₂: 57% by moles
   CaO: 34% by moles
   Na₂O: 6% by moles
   Al₂O₃: 3% by moles
   Particle size: below 20 µm
   Specific surface: a few m²/g
   Almost zero porosity
   Liquid to powder ratio ranges between 0,5 ml/g and 0,6 ml/g.

## Claims

1. An injectable composition for bone filling or consolidation, for use in surgery or therapy, **characterized in that** it comprises a biocompatible material in form of powder, which is hardenable or cross-linkable and selected from calcium sulphate hemi-hydrate and polymethylmethacrylate, a hardening or cross-linking agent for said material and a porous silica-based material in form of powder, having a specific surface area higher than 100 m²/g, which comprises a mesoporous bioactive glass having a pore size from 2 to 50 nm, obtainable by a sol-gel method with the addition of a surfactant as structure-directing agent for pores formation, comprising an oxide mixture containing:
SiO₂: from 60 to 85% by moles
CaO: from 10 to 30% by moles
P₂O₅: from 2 to 10% by moles
said composition, when applied to an affected area of a patient, being adapted to form a composite material wherein said hardenable or cross-linkable material constitutes a matrix and said mesoporous bioactive glass constitutes a dispersed phase in said matrix.

2. A composition according to claim 1, **characterized in that** it comprises a radio-opaque mesoporous bioactive glass comprising ZrO₂ or SrO or mixtures thereof in an amount from 2 to 15% by moles referred to 100 % by moles of the mixture of SiO₂, CaO and P₂O₅ oxides.

3. A composition according to claims 1 or 2, **characterized in that** said mesoporous bioactive glass is a powder with particles size ranging between 5 and 32 µm as obtainable through sieving.

4. A composition according to any of claims 1 to 3, comprising a further dispersed phase, of glassy nature, in the form of a powder or fibres based on SiO₂ and CaO, with the optional addition of one or more oxides selected from the group consisting of Na₂O, MgO, K₂O, Al₂O₃, ZrO₂, SrO and TiO₂ and mixtures thereof.

5. A composition according to any of claims 1 to 4, further comprising an additional bio-resorbable dispersed phase in the form of powders or fibres, based on P₂O₅ and CaO, containing one or more oxides selected from the group consisting of Na₂O, MgO, K₂O and TiO₂ and mixtures thereof.

6. A composition according to any of claims 1 to 5, **characterized in that** the matrix material comprises powders of α-calcium sulphate hemi-hydrate and **in that** it further comprises an agent delaying the onset of the hardening, such as carboxylic acids selected from tartaric acid, citric acid, succinic acid, malic acid and mixtures thereof.

7. A composition according to claim 6, comprising as matrix material a calcium phosphate or calcium phosphates, in combination with calcium sulphate hemi-hydrate.

8. A composition according to any of the preceding claims, **characterized in that** it comprises from 40% to 95% by weight, preferably from 60% to 90% by weight of matrix material, referred to 100% by weight of the matrix material and dispersed phases.

9. A composition according to any of the preceding claims, comprising an aqueous carrier selected from distilled water, balanced saline solution, optionally containing a radio-opaque agent.

10. A composition according to claim 9, further comprising a biocompatible organic oil.

11. A composition according to any of the preceding claims, **characterized in that** said mesoporous bioactive glass is loaded with biologically active substances, selected from antibiotic drugs, anti-inflammatories, painkillers, anti-tumorals and growth factors, particularly morphogenetic proteins.

12. A composition according to any of the preceding claims, **characterized in that** said mesoporous bioactive glass has a specific surface area comprised between 300 m²/g and 500 m²/g and a pore dimension/size comprised between 2 nm and 10 nm.

13. A composite bone cement obtained from a composition according to any of claims 1 to 12.

## Patentansprüche

1. Injizierbare Zusammensetzung zur Knochenfüllung oder Konsolidierung zur Verwendung in Chirurgie oder Therapie, **dadurch gekennzeichnet,**
**dass** es ein biokompatibles Material in Form eines Pulvers, welches aushärtbar oder vernetzbar und ausgewählt ist aus Calciumsulfat-Halbhydrat und
Polymethylmethacrylat, ein Aushärtungs- oder Vernetzungsmittel für das Material und ein poröses Siliziumdioxid-basiertes Material in Form eines Pulvers umfasst, welches eine innere Oberfläche von mehr als 100 m²/g aufweist, welche ein mesoporöses bioaktives Glas umfasst, welches eine Porengröße von 2 bis 50 nm aufweist, welches durch ein Sol-Gel-Verfahren unter Zugabe eines grenzflächenaktiven Stoffs als einem Strukturdirigenten für eine Bildung von Poren, umfassend ein Oxidgemisch, welches enthält:
SiO₂: von 60 bis 85 Mol-%
CaO: von 10 bis 30 Mol-%
P₂O₅: von 2 bis 10 Mol-%
wobei die Zusammensetzung, wenn sie an einem betroffenen Bereich eines Patienten angewendet wird, dazu eingerichtet ist, ein Verbundmaterial zu bilden, wobei das aushärtbare oder vernetzbare Material eine Matrix ausbildet, und wobei das mesoporöse bioaktive Glas eine dispergierte Phase in der Matrix ausbildet.

2. Zusammensetzung nach Anspruch 1, **dadurch gekennzeichnet, dass** sie ein strahlenundurchlässiges mesoporöses bioaktives Glas umfasst, umfassend ZrO₂ oder SrO oder Mischungen davon in einer Menge von 2 bis 15 Mol-% bezogen auf 100% mol der Mischung aus SiO₂, CaO und P₂O₅ Oxiden.

3. Zusammensetzung nach Anspruch 1 oder 2, **dadurch gekennzeichnet, dass** das mesoporöse bioaktive Glas ein Pulver mit einer zwischen 5 und 32 µm liegenden Partikelgröße ist, erhältlich durch Sieben.

4. Zusammensetzung nach einem der Ansprüche 1 bis 3, umfassend eine weitere dispergierte Phase mit einer glasartigen Beschaffenheit in Form eines Pulvers oder von Fasern auf Basis von SiO₂ und CaO, mit dem optionalen Zusatz von einem oder mehreren Oxiden, welche aus der aus Na₂O, MgO, K₂O, Al₂O₃, ZrO₂, SrO und TiO₂ und Gemischen davon bestehenden Gruppe ausgewählt sind.

5. Zusammensetzung nach einem der Ansprüche 1 bis 4, ferner umfassend eine zusätzliche biologisch resorbierbare dispergierte Phase in der Form von Pulvern oder Fasern auf Basis von P₂O₅ und CaO, enthaltend ein oder mehrere Oxide, welche aus der aus Na₂O, MgO, K₂O und TiO₂ und Mischungen davon bestehenden Gruppe ausgewählt sind.

6. Zusammensetzung nach einem der Ansprüche 1 bis 5, **dadurch gekennzeichnet, dass** das Matrixmaterial ein Pulver von α-Calciumsulfat-Halbhydrat umfasst und dass es ferner ein Mittel zur Verzögerung des Beginns der Härtung, wie Carbonsäuren, umfasst, ausgewählt aus Weinsäure, Zitronensäure, Bernsteinsäure, Äpfelsäure und Mischungen davon.

7. Zusammensetzung nach Anspruch 6, umfassend in Kombination mit Calciumsulfat-Halbhydrat als ein Matrixmaterial ein Calciumphosphat oder Calciumphosphate.

8. Zusammensetzung nach einem der vorhergehenden Ansprüche, **dadurch gekennzeichnet, dass** sie 40 Gew.-% bis 95 Gew.-%, vorzugsweise 60 Gew.-% bis 90 Gew.-% von Matrixmaterial bezogen auf 100 Gew.-% des Matrixmaterials und von dispergierten Phasen umfasst.

9. Zusammensetzung nach einem der vorhergehenden Ansprüche, umfassend einen wässrigen Träger ausgewählt aus destilliertem Wasser, einem Mineralsalzmedium, welcher optional ein strahlenundurchlässiges Mittel enthält.

10. Zusammensetzung nach Anspruch 9, ferner umfassend ein biokompatibles organisches Öl.

11. Zusammensetzung nach einem der vorhergehenden Ansprüche, **dadurch gekennzeichnet, dass** das mesoporöse bioaktive Glas mit biologisch aktiven Substanzen beladen ist, welche aus Antibiotika, entzündungshemmenden Mitteln, Schmerzmitteln, gegen Tumore wirksamen Mitteln und Wachstumsfaktoren, insbesondere morphogenetischen Proteinen, ausgewählt sind.

12. Zusammensetzung nach einem der vorhergehenden Ansprüche, **dadurch gekennzeichnet, dass** das mesoporöse bioaktives Glas eine zwischen 300 m²/g und 500 m²/g liegende innere Oberfläche und eine zwischen 2 nm und 10 nm liegende Porendimension/ -größe aufweist.

13. Ein aus einer Zusammensetzung nach einem der Ansprüche 1 bis 12 erhaltener Verbundknochenzement.

## Revendications

1. Composition injectable pour remplissage ou consolidation d'un os, pour utilisation en chirurgue ou en thérapie, **caractérisée en ce qu'**elle comprend un matériau biocompatible, sous forme de poudre, qu'on peut faire durcir ou réticuler et qui est choisi parmi du sulfate de calcium hémi-hydraté et du poly(méthacrylate de méthyle), un durcisseur ou un agent de réticulation pour ledit matériau, et un matériau poreux à base de silice, sous forme de poudre, qui présente une aire spécifique de plus de 100 m²/g et qui comprend un verre bioactif mésoporeux dont les pores ont une taille de 2 à 50 nm, accessible par un procédé sol-gel avec ajout d'un tensioactif en tant qu'agent structurant pour formation de pores, et comprenant un mélange d'oxydes qui contient
- de 60 à 85 % en moles de SiO₂,
- de 10 à 30 % en moles de CaO,
- et de 2 à 10 % en moles de P₂O₅,
laquelle composition est adaptée pour former, lorsqu'elle est appliquée à une zone atteinte d'un patient, un matériau composite dans lequel ledit matériau durcissable ou réticulable constitue une matrice et ledit verre bioactif mésoporeux constitue une phase dispersée au sein de ladite matrice.

2. Composition conforme à la revendication 1, **caractérisée en ce qu'**elle comprend un verre bioactif mésoporeux et radio-opaque, comprenant du ZrO₂, du SrO ou un mélange de ces oxydes, en une proportion de 2 à 15 % en moles, rapportée à 100 % moles du mélange des oxydes SiO₂, CaO et P₂O₅.

3. Composition conforme à la revendication 1 ou 2, **caractérisée en ce que** ledit verre bioactif mésoporeux est une poudre dont les particules on une taille qui vaut de 5 à 32 µm, accessible par tamisage.

4. Composition conforme à l'une des revendications 1 à 3, comprenant une phase dispersée supplémentaire, de nature vitreuse, sous la forme d'une poudre ou de fibres à base de SiO₂ et de CaO, avec, en option, ajout d'un ou de plusieurs oxyde(s) choisi(s) dans l'ensemble constitué par Na₂O, MgO, K₂O, Al₂O₃, ZrO₂, SrO et TiO₂, ainsi que leurs mélanges.

5. Composition conforme à l'une des revendications 1 à 4, comprenant en outre une phase dispersée additionnelle bio-résorbable, sous la forme de poudres ou de fibres, à base de P₂O₅ et de CaO, contenant un ou plusieurs oxyde(s) choisi(s) dans l'ensemble constitué par Na₂O, MgO, K₂O et TiO₂, ainsi que leurs mélanges.

6. Composition conforme à l'une des revendications 1 à 5, **caractérisée en ce que** le matériau de matrice comprend de l'hémi-hydrate alpha de sulfate de calcium en poudre et **en ce qu'**elle comprend en outre un agent retardant le démarrage du durcissement, tels des acides carboxyliques choisis parmi l'acide tartrique, l'acide citrique, l'acide succinique et l'acide malique ainsi que leurs mélanges.

7. Composition conforme à la revendication 6, comprenant en tant que matériau de matrice, en combinaison avec l'hémi-hydrate de sulfate de calcium, un phosphate de calcium ou des phosphates de calcium.

8. Composition conforme à l'une des revendications précédentes, **caractérisée en ce qu'**elle comprend de 40 à 95 % en poids, et de préférence de 60 à 90 % en poids, de matériau de matrice pour 100 % en poids du matériau de matrice et des phases dispersées.

9. Composition conforme à l'une des revendications précédentes, comprenant un véhicule aqueux choisi parmi de l'eau distillée et une solution salée équilibrée, et contenant, en option, un agent radio-opaque.

10. Composition conforme à la revendication 9, comprenant en outre une huile organique biocompatible.

11. Composition conforme à l'une des revendications précédentes, **caractérisée en ce que** ledit verre bioactif mésoporeux est chargé de substances possédant une activité biologique, choisies parmi les médicaments antibiotiques, anti-inflammatoires, antidouleurs et anti-tumoraux et les facteurs de croissance, en particulier les protéines morphogénétiques.

12. Composition conforme à l'une des revendications précédentes, **caractérisée en ce que** ledit verre bioactif mésoporeux présente une aire spécifique valant de 300 à 500 m²/g et des pores de taille ou dimension valant de 2 à 10 nm.

13. Ciment osseux composite, obtenu à partir d'une composition conforme à l'une des revendications 1 à 12.
